# FASCICULE DE BREVET EUROPEEN

(11) **EP 0 706 790 B1**
(45) Date de publication et mention de la délivrance du brevet: **30.07.1997**
(21) Numéro de dépôt: 95402029.3
(22) Date de dépôt: 07.09.1995
(51) Int. Cl.: A61K 7/48, A61K 7/025, A61K 7/027

(54) **Composition cosmétique anhydre sous forme de pâte souple et procédé de préparation**
Wasserfreie kosmetische Zusammensetzung in Form einer weichen Pasta und Herstellungsverfahren
Anhydrous cosmetic composition in form of a supple paste and preparation process

(30) Priorité: 06.10.1994 FR 9411946
(43) Date de publication de la demande: 17.04.1996
(73) Titulaire: L'OREAL, 75008 Paris (FR)
(72) Inventeur: Le Bras, Véronique, F-75002 Paris (FR); Miguel, Dolorès, F-92340 Bourg la Reine (FR)
(74) Mandataire: Dodin, Catherine

(56) Documents cités:
- EP-A- 0 602 905
- US-A- 5 085 855

## Description

La présente invention a trait à une composition, par exemple cosmétique ou pharmaceutique, se présentant sous forme d'une pâte souple, pouvant être utilisée pour le maquillage, en particulier des lèvres, ou comme base pour le traitement de la peau et/ou des lèvres.

Les compositions cosmétiques pouvant être appliquées sur les lèvres comme produit de maquillage ou de soin, tels les rouges à lèvres par exemple, contiennent généralement des corps gras et des cires, et éventuellement des additifs et des pigments.
Il est connu que plus la quantité de cires présente dans la composition est importante, plus celle-ci a une consistance ferme, ce qui permet son utilisation sous forme de bâton.
Or la présentation, en particulier d'un rouge à lèvres, sous forme de bâton présente certains inconvénients: le dessin des contours des lèvres est malaisé et la tenue du bâton à la chaleur n'est pas optimale.
Afin d'obtenir une composition cosmétique de texture plus souple, il a été proposé d'introduire dans la formulation des gommes qui permettent de conférer à la composition finale des qualités de douceur et de glissant remarquables, tout en lui évitant d'être huileuse. Parmi les gommes pouvant être incorporées dans ces compositions, les gommes de silicone se sont révélées particulièrement intéressantes car permettant d'obtenir la texture désirée.
Toutefois, leur introduction dans une composition riche en corps gras, telle qu'un rouge à lèvres, pose problème.
En effet, lorsque l'on prépare un mélange comprenant des corps gras tels qu'habituellement utilisés dans l'industrie cosmétique, et des gommes de silicone, on observe généralement l'apparition de deux phases qui résultent du fait que les gommes de silicone ne peuvent pas être mélangées de façon homogène avec des corps gras lorsque l'on utilise les techniques de l'art antérieur.
Pour pallier cet inconvénient, plusieurs solutions sont envisageables, parmi lesquelles on peut citer:
- la présolubilisation des gommes de silicone dans une huile solubilisante à bas point d'ébullition telle qu'une huile de silicone; cette solution n'est toutefois pas satisfaisante car des problèmes de conservation peuvent se poser, en particulier dus à l'évaporation des silicones volatils.
- l'utilisation de corps gras siliconés exclusivement; cette solution permet bien l'obtention d'un mélange homogène mais l'introduction d'un corps gras non siliconé conduit inévitablement à une séparation de phase. - l'utilisation d'esters courts tels que le myristate d'isopropyle, corps gras compatible à la fois avec les produits siliconés et les corps gras non siliconés; toutefois l'utilisation de cet ester peut entraîner une certaine sensibilisation de la muqueuse.

La présente invention a pour but de pallier les inconvénients de l'art antérieur et de proposer un procédé permettant l'obtention d'une composition cosmétique anhydre comprenant des gommes de silicone et des corps gras, siliconés et/ou non siliconés, stable et homogène.

Un objet de la présente invention est donc une composition anhydre à usage topique se présentant sous forme d'une pâte souple, comprenant une gomme de silicone et des corps gras dont au moins une cire.
Un autre objet de l'invention est un procédé de préparation d'une composition anhydre se présentant sous forme d'une pâte souple et comprenant une gomme de silicone et des corps gras dont au moins une cire, dans lequel on porte à une température à laquelle la cire fond, au moins une partie des différents constituants de la composition dont la cire, on ajoute le cas échéant le reste des constituants, puis l'on malaxe le mélange obtenu pendant au moins une partie de son refroidissement.

On a constaté que le procédé selon l'invention permet d'obtenir des compositions cosmétiques comprenant des corps gras, siliconés ou non, et des gommes de silicone, présentant une grande homogénéité, sans nécessiter l'incorporation d'un composé particulier pour maintenir la stabilité desdites compositions.
La présente invention a donc pour avantage de permettre de varier les formulations des compositions sans être tenu par la présence obligatoire d'un certain type de composés, tout en obtenant des compositions qui restent homogènes et stables dans le temps.
On a de plus constaté que, de manière surprenante, les compositions selon l'invention possèdent une texture souple originale et nouvelle, et présentent, après application, une bonne tenue et une bonne brillance.

Dans la suite de la présente description, les pourcentages sont donnés en poids sauf indication contraire.

La composition selon la présente invention comprend donc une gomme de silicone et des corps gras dont au moins une cire.

La gomme de silicone peut être présente généralement en une quantité de l'ordre de 0,1-10%, de préférence de 0,1 à 3% par rapport au poids de la composition finale.

On utilise de préférence, seule ou en mélange, une gomme de silicone ayant un poids moléculaire inférieur à 1 500 000 telle qu'un polydiméthylsiloxane, un polyphénylsiloxane ou un polyhydroxysiloxane.
En particulier, on peut utiliser une gomme de silicone répondant à la formule : 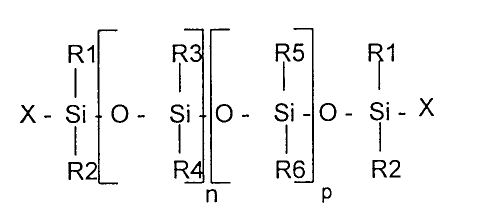 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
De manière générale, n et p peuvent prendre des valeurs de 0 à 5000, de préférence de 0 à 3000.
La gomme de silicone peut être introduite dans la composition telle quelle ou sous forme diluée dans une huile de silicone telle qu'un PDMS (polydiméthylsiloxane).
Comme gomme de silicone utilisable selon l'invention, on peut citer celles pour lesquelles :
. les substituants R₁ à R₆ et X représentent un groupement méthyle, p = 0 et n = 2700, comme celle vendue sous la dénomination SE30 par la société Général Electric,
. les substituants R₁ à R₆ et X représentent un groupement méthyle, p = 0 et n = 2300, comme celle vendue sous la dénomination AK 500000 par la société Waker,
. les substituants R₁ à R₆ représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R₁ à R₆ représentent un groupement méthyle, le substituant X représente un groupement hydroxyle, p = 0 et n = 2700, en solution à 13% dans le diméthicone, comme celle vendue sous la dénomination Q2-1403 par la société Dow Corning,
. les substituants R₁, R₂ R₅, R₆ et X représentent un groupement méthyle, les substituants R₃ et R₄ représentent un groupement aryle tel que le poids moléculaire du composé soit de 600 000, comme celle vendue sous la dénomination 761 par la société Rhône-Poulenc.

La composition selon l'invention comprend également des corps gras, dont au moins une cire.
Dans une forme particulière de l'invention, au moins une partie des corps gras est constituée de corps gras non siliconés.
En tant que corps gras, on entend peut utiliser des composés sous forme de cires, d'huiles ou de pâteux.
Parmi les cires susceptibles d'être utilisées en tant que corps gras non siliconés, on peut citer les cires animales telles que la cire d'abeilles; les cires végétales telles que la cire de Carnauba, de Candellila, d'Ourrury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.
Parmi les cires siliconées, on peut citer les alkyls, alkoxys et/ou esters de polyméthylsiloxane.
Les cires peuvent être présentes en une quantité de 2-50%, de préférence 10-30%, par rapport au poids de la composition finale.

Parmi les éventuels autres corps gras non siliconés, on peut citer les huiles minérales telles que l'huile de paraffine ou de vaseline, les huiles animales telles que le perhydrosqualène ou l'huile d'arara, ou encore les huiles végétales telles que l'huile d'amande douce, de calophyllum, de palme, de ricin, d'avocat, de jojoba, d'olive ou de germes de céréales.
On peut également utiliser des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools.
On peut encore utiliser des huiles hydrogénées concrètes à 25°C telles que les huiles de ricin, de palme ou de coco hydrogénées, ou le suif hydrogéné; des mono-, di- , tri- ou sucro- glycérides; des lanolines; des esters gras concrets à 25°C.

On peut ajouter à ladite composition un agent de coloration pulvérulent tel que le noir de carbone, les oxydes de chrome ou de fer, les outremers, le pyrophosphate de manganèse, le bleu ferrique, le dioxyde de titane, les agents nacrants généralement utilisés en mélange avec des pigments colorés ou certains colorants organiques généralement utilisés en mélange avec des pigments colorés et couramment utilisés dans l'industrie cosmétique. Ces agents de coloration peuvent être présents en une quantité de 0 à 20%.

On peut également ajouter des charges pulvérulentes minérales ou organiques, généralement en une quantité de 0 à 40%.
Ces charges pulvérulentes peuvent être choisies parmi le talc, les micas, le kaolin, les oxydes de zinc ou de titane, les carbonates de calcium ou de magnésium, la silice, le dioxyde de titane sphérique, les billes de verre ou de céramiques, les savons métalliques dérivés d'acides carboxyliques ayant 8-22 atomes de carbone, les poudres de polymères synthétiques non expansés, les poudres expansées et les poudres de composés organiques naturels tels que les amidons de céréales, réticulés ou non.

On peut encore incorporer dans ladite composition tout additif usuellement utilisé dans l'industrie cosmétique, tel que des antioxydants, des parfums, des conservateurs, ainsi que des actifs cosmétiques et/ou pharmaceutiques tels que des dérivés de vitamines, des acides gras essentiels, des sphingocérils ou des filtres solaires liposolubles, par exemple.

Afin de préparer la composition selon l'invention, on prépare tout d'abord un prémélange comprenant au moins une partie des différents constituants de la composition, dont au moins la ou les cires, on chauffe ce prémélange à une température à laquelle la cire fond, on ajoute le cas échéant le reste des constituants, puis l'on malaxe le mélange obtenu pendant au moins une partie de son refroidissement jusqu'à température ambiante.

On a en effet constaté que ce procédé permet d'obtenir une composition se présentant sous forme de pâte souple et homogène, bien qu'elle contienne des corps gras et une gomme de silicone.
On peut effectuer l'opération de chauffage selon toute technique connue.
L'opération de malaxage peut être effectuée grâce à une agitation énergique ou par extrusion.
Dans un mode particulier de réalisation de l'invention, les opérations de chauffage et de malaxage, voire de refroidissement, sont réalisées dans un ou plusieurs extrudeurs disposés à la suite les uns des autres, et de préférence dans un extrudeur bivis unique.
On a en effet constaté que la composition obtenue après extrusion présente une douceur particulière, et procure une certaine sensation de glissant lorsqu'elle est appliquée sur la peau, tout en évitant l'aspect et la sensation de gras huileux.
Les conditions dans laquelle l'extrusion peut être effectuée sont décrites dans la demande de brevet FR94-00756 dont le contenu est incorporé à la présente demande par référence.

On obtient ainsi une composition anhydre à usage topique, qui peut être appliquée sur la peau et/ou sur les lèvres en tant que produit de maquillage, rouge à lèvres par exemple, et/ou de produit de soin.
Selon l'utilisation envisagée, la composition peut comprendre les constituants habituellement utilisés par l'homme du métier.

L'invention est illustrée plus en détail dans les exemples suivants.

### Exemple 1

On prépare une base traitante pour lèvres ayant la formulation suivante:

| | |
|---|---|
| . huile de vaseline | 22% |
| . lanoline | 23% |
| . lanolate d'isopropyle | 23% |
| . cire microcristalline | 15% |
| . cire de Carnauba | 10% |
| . butylhydroxytoluène | 0,2% |
| . acétate de tocophérol | 1,8% |
| . diméthiconol en solution à 13% dans diméthicone (Q2 1403 de Dow Corning) | 5 % |

On mélange ces différents ingrédients à environ 100°C, et l'on introduit le mélange dans un extrudeur bi-vis.

L'extrusion est effectuée dans les conditions suivantes:
. température d'entrée : 100°C
. température de sortie : 30°C
. temps de résidence : 3 minutes environ
. vitesse des vis : 350 tr/min

On obtient en sortie une pâte souple de viscosité égale à 15 Pa.s, se présentant sous forme d'une seule phase stable et homogène, et pouvant être prélevée à l'aide d'un pinceau pour son application.
Après application, cette base traitante est considérée comme présentant des qualités de douceur et de glissant satisfaisantes, et ne présente pas de texture huileuse.

### Exemple 2

On prépare un rouge à lèvres de formulation suivante:

| | |
|---|---|
| . huile de jojoba | 11% |
| . huile de ricin | 25% |
| . lanoline | 10% |
| . cire d'abeilles | 15% |
| . cire de polyéthylène | 17% |
| . polybutylène | 4% |
| . pigment D&C Yellow no 6 (Cl 15985) | 1% |
| . pigment D&C Red no 27 (Cl 45410) | 9% |
| . dioxyde de titane (Cl 77891) | 3% |
| . polyphénylsiloxane (Silbione 71634 de R. Poulenc) | 5% |

On broie les pigments dans les constituants gras (huiles et lanoline) puis l'on ajoute les autres constituants et l'on chauffe le mélange à 100°C. On procède de manière similaire à celle de l'exemple 1, et l'on obtient un rouge à lèvre de viscosité égale à 16 Pa.s, présentant des qualités de douceur et de glissant satisfaisantes.

### Exemple 3

On prépare un rouge à lèvres de formulation suivante:

| | |
|---|---|
| . huile de jojoba | 20% |
| . huile de vaseline | 20% |
| . lanoline | 21% |
| . talc | 5% |
| . poudre de Nylon | 5% |
| . cire de Candelilla | 7% |
| . cire de polyéthylène | 10% |
| . pigment D&C Yellow 6 Aluminium Lake (Cl 15985) | 5% |
| . pigment D&C Red 7 (Cl 5850:1) | 5,8% |
| . oxyde de fer (Cl 77499) | 0,2% |
| . polydiméthylsiloxanol | 1% |

On broie les pigments dans les constituants gras (huiles et lanoline) puis l'on ajoute les autres constituants et l'on chauffe le mélange à 100°C.
On introduit ensuite le mélange dans un extrudeur bi-vis, et l'on extrude dans les même conditions qu'à l'exemple 1.
On obtient un rouge à lèvres de viscosité égale à 10 Pa.s, présentant des qualités de glissant et de douceur satisfaisantes ainsi qu'une certaine brillance.

## Revendications

1. Composition anhydre à usage topique se présentant sous forme d'une pâte souple, comprenant une gomme de silicone et des corps gras dont au moins une cire.

2. Composition selon la revendication 1, dans laquelle la gomme de silicone est présente en une quantité de 0,1-10%, de préférence de 0,1 à 3% par rapport au poids de la composition finale.

3. Composition selon l'une des revendications précédentes, dans laquelle la gomme de silicone est choisie parmi les gommes de silicone ayant un poids moléculaire inférieur à 1 500 000 telles qu'un polydiméthylsiloxane, un polyphénylsiloxane ou un polyhydroxysiloxane, et/ou parmi les gommes répondant à la formule : 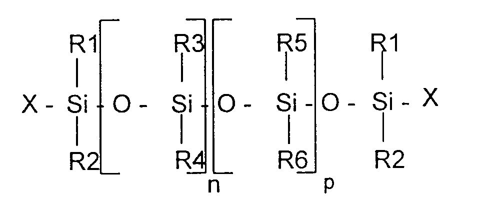 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

4. Composition selon l'une des revendications précédentes, dans laquelle au moins une partie des corps gras est constituée de corps gras non siliconés.

5. Composition selon l'une des revendications précédentes, dans laquelle la cire est choisie parmi les cires animales telles que la cire d'abeilles; les cires végétales telles que la cire de Carnauba, de Candellila, d'Ourrury, du Japon, les cires de fibres de lièges ou de canne à sucre; les cires minérales telles que les cires de paraffine, de lignite, les cires microcristallines, les ozokérites; les cires synthétiques, telles que les cires de polyéthylène et les cires obtenues par synthèse de Fischer-Tropsch; les cires siliconées telles que les alkyls, alkoxys et/ou esters de polyméthylsiloxane.

6. Composition selon l'une des revendications précédentes, dans laquelle la cire est présente en une quantité de 2-50%, de préférence 10-30%, par rapport au poids de la composition finale.

7. Composition selon l'une des revendications précédentes, dans laquelle les corps gras sont choisis parmi les huiles minérales telles que l'huile de paraffine ou de vaseline; les huiles animales telles que le perhydrosqualène ou l'huile d'arara; les huiles végétales telles que l'huile d'amande douce, de calophyllum, de palme, de ricin, d'avocat, de jojoba, d'olive ou de germes de céréales; les esters d'acide lanolique, d'acide oléique, d'acide laurique, d'acide stéarique ou d'acide myristique; les alcools tels que l'alcool oléique, l'alcool linoléique ou linolénique, l'alcool isostéarique ou l'octyl dodécanol; les acétylglycérides, les octanoates, décanoates ou ricinoléates d'alcools ou de polyalcools; les huiles hydrogénées concrètes à 25°C telles que les huiles de ricin, de palme ou de coco hydrogénées, ou le suif hydrogéné; les mono-, di- , tri- ou sucro- glycérides; les lanolines; les esters gras concrets à 25°C.

8. Procédé de préparation d'une composition anhydre se présentant sous forme d'une pâte souple et comprenant une gomme de silicone et des corps gras dont au moins une cire, dans lequel on porte à une température à laquelle la cire fond, au moins une partie des différents constituants de la composition dont la cire, on ajoute le cas échéant le reste des constituants, puis l'on malaxe le mélange obtenu pendant au moins une partie de son refroidissement.

9. Procédé selon la revendication 8, dans lequel l'opération de malaxage est réalisée dans au moins un extrudeur.

10. Procédé selon la revendication 8, dans lequel les opérations de mélange, de chauffage et de malaxage sont réalisées dans un extrudeur bi-vis unique.

## Claims

1. Anhydrous composition for topical use, which is provided in the form of a soft paste, comprising a silicone gum and fatty substances, including at least one wax.

2. Composition according to Claim 1, in which the silicone gum is present in an amount of 0.1-10 %, preferably from 0.1 to 3 %, relative to the weight of the final composition.

3. Composition according to either of the preceding claims, in which the silicone gum is chosen from silicone gums having a molecular weight of less than 1,500,000, such as a polydimethylsiloxane, a polyphenylsiloxane or a polyhydroxysiloxane, and/or from the gums corresponding to the formula: 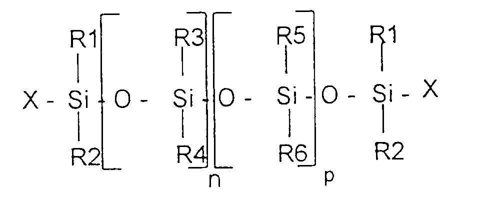 in which:
R₁, R₂, R₅ and R₆ are, together or separately, an alkyl radical having 1 to 6 carbon atoms,
R₃ and R₄ are, together or separately, an alkyl radical having from 1 to 6 carbon atoms or an aryl radical,
X is an alkyl radical having from 1 to 6 carbon atoms, a hydroxyl radical or a vinyl radical,
n and p being chosen so as to give the silicone gum a viscosity greater than 100,000 mPa.s, preferably greater than 500,000 mPa.s.

4. Composition according to one of the preceding claims, in which at least a portion of the fatty substances consists of non-silicone fatty substances.

5. Composition according to one of the preceding claims, in which the wax is chosen from animal waxes, such as beeswax; plant waxes, such as carnauba, candelilla, ouricurry or Japan wax, cork fibre wax or sugarcane wax; mineral waxes, such as paraffin wax or lignite wax, microcrystalline waxes or ozokerites; synthetic waxes, such as polyethylene waxes and the waxes obtained by Fischer-Tropsch synthesis; or silicone waxes, such as polymethyl-siloxane alkyls, alkoxys and/or esters.

6. Composition according to one of the preceding claims, in which the wax is present in an amount of 2-50 %, preferably 10-30 %, relative to the weight of the final composition.

7. Composition according to one of the preceding claims, in which the fatty substances are chosen from mineral oils, such as liquid paraffin or liquid petrolatum; animal oils, such as perhydrosqualene or arara oil; plant oils, such as sweet almond oil, calophyllum oil, palm oil, castor oil, avocado oil, jojoba oil, olive oil or cereal germ oil; esters of lanolic acid, of oleic acid, of lauric acid, of stearic acid or of myristic acid; alcohols, such as oleyl alcohol, linoleyl or linolenyl alcohol, isostearyl alcohol or octyldodecanol; acetylglycerides, octanoates, decanoates or ricinoleates of alcohols or of polyalcohols; hydrogenated oils which are solid at 25°C, such as hydrogenated castor, palm or coconut oils, or hydrogenated tallow; mono-, di-, tri- or sucroglycerides; lanolins; or fatty esters which are solid at 25°C.

8. Process for the preparation of an anhydrous composition which is provided in the form of a soft paste and which comprises a silicone gum and fatty substances, including at least one wax, in which at least a portion of the various constituents of the composition, including the wax, is brought to a temperature at which the wax melts, the remainder of the constituents are added, if appropriate, and the mixture obtained is then kneaded during at least a part of its cooling step.

9. Process according to Claim 8, in which the kneading operation is carried out in at least one extruder.

10. Process according to Claim 8, in which the mixing, heating and kneading operations are carried out in a single twin-screw extruder.

## Patentansprüche

1. Wasserfreie Zusammensetzung zur topischen Anwendung, die in Form einer weichen Paste vorliegt und einen Silicongummi und Fettsubstanzen und darunter mindestens ein Wachs enthält.

2. Zusammensetzung nach Anspruch 1, wobei der Silicongummi in einem Mengenanteil von 0,1 bis 10 % und vorzugsweise von 0,1 bis 3 %, bezogen auf das Gewicht der am Ende vorliegenden Zusammensetzung, vorliegt.

3. Zusammensetzung nach einem der vorhergehenden Ansprüche, wobei der Silicongummi unter den Silicongummis mit einem Molekulargewicht unter 1 500 000, wie Polydimethylsiloxanen, Polyphenylsiloxanen oder Polyhydroxysiloxanen, und/oder unter den Gummis der Formel 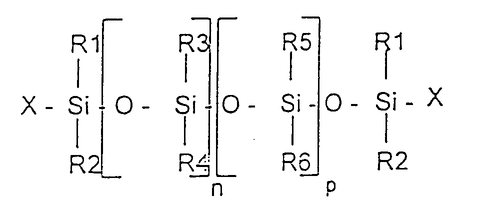 ausgewählt ist, wobei in der Formel bedeuten:
R₁, R₂, R₅ und R₆ gleichzeitig oder unabhängig voneinander eine Alkylgruppe mit 1 bis 6 Kohlenstoffatomen,
R₃ und R₄ gleichzeitig oder unabhängig voneinander eine Alkylgruppe mit 1 bis 6 Kohlenstoffatomen oder eine Arylgruppe,
X eine Alkylgruppe mit 1 bis 6 Kohlenstoffatomen, eine Hydroxygruppe oder eine Vinylgruppe,
wobei n und p so ausgewählt sind, daß der Silicongummi eine Viskosität über 100000 mPa·s und vorzugsweise über 500000 mPa·s aufweist.

4. Zusammensetzung nach einem der vorhergehenden Ansprüche, wobei mindestens ein Teil der Fettsubstanzen aus nichtsiliconhaltigen Fettsubstanzen besteht.

5. Zusammensetzung nach einem der vorhergehenden Ansprüche, wobei das Wachs unter den tierischen Wachsen, wie Bienenwachs, den pflanzlichen Wachsen, wie Carnaubawachs, Candellilawachs, Ouricuriwachs, Japanwachs und den Wachsen von Korkfasern oder Zuckerrohrfasern, den mineralischen Wachsen, wie Paraffinwachs, Lignitwachs, mikrokristallinen Wachsen oder Ozokeriten, synthetischen Wachsen, wie Polyethylenwachsen und Wachsen, die durch Fischer-Tropsch-Synthese hergestellt sind, und Siliconwachsen, wie den Alkyl-, Alkoxy- und/oder Esterverbindungen von Polydimethylsiloxan, ausgewählt ist.

6. Zusammensetzung nach einem der vorhergehenden Ansprüche, wobei das Wachs in einem Mengenanteil von 2 - 50 % und vorzugsweise von 10 - 30 %, bezogen auf das Gewicht der am Ende vorliegenden Zusammensetzung, vorliegt.

7. Zusammensetzung nach einem der vorhergehenden Ansprüche, wobei die Fettsubstanzen unter den mineralischen Ölen, wie Paraffinöl oder Vaselineöl, tierischen Ölen, wie Perhydrosqualen oder Araraöl, den pflanzlichen Ölen, wie süßem Mandelöl, Calophyllumöl, Palmöl, Ricinusöl, Avocadoöl, Jojobaöl, Olivenöl oder Ölen von Cerealienkeimen, Estern von Lanolinsäure, Ölsäure, Laurinsäure, Stearinsäure oder Myristinsäure, Alkoholen, wie Oleylalkohol, Linoleylalkohol, Linolenylalkohol, Isostearylalkohol oder Octyldodecanol, Acetylglyceriden, Octanoaten, Decanoaten oder Ricinoleaten von Alkoholen oder Polyalkoholen, bei 25 °C festen hydrierten Ölen, wie hydriertem Ricinusol, Palm- oder Kokosöl oder hydriertem Talk, Mono-, Di-, Tri- oder Sucroglyceriden, Lanolinen und bei 25 °C festen Fettsäureestern ausgewählt sind.

8. Verfahren zur Herstellung einer wasserfreien Zusammensetzung, die in Form einer weichen Paste vorliegt und einen Silicongummi und Fettsubstanzen und darunter mindestens ein Wachs enthält, wobei mindestens ein Teil der verschiedenen Bestandteile der Zusammensetzung und darunter das Wachs auf eine Temperatur erwärmt wird, bei der das Wachs schmilzt, ggf. die übrigen Bestandteile zugegeben und anschließend das hergestellte Gemisch während zumindest einem Teil der zum Abkühlen benötigten Zeitspanne geknetet wird.

9. Verfahren nach Anspruch 8, wobei der Arbeitsgang des Knetens in mindestens einem Extruder durchgeführt wird.

10. Verfahren nach Anspruch 8, wobei die Arbeitsgänge des Mischens, Erwärmens und Knetens in einem einzigen Zweischneckenextruder durchgeführt werden.
